# EUROPÄISCHE PATENTSCHRIFT

(11) **EP 3 282 243 B1**
(45) Veröffentlichungstag und Bekanntmachung des Hinweises auf die Patenterteilung: **09.01.2019**
(21) Anmeldenummer: 16183750.5
(22) Anmeldetag: 11.08.2016
(51) Int. Cl.: G01N 21/3554, G01N 21/84, G01N 21/3563

(54) **VERFAHREN UND MESSSYSTEM ZUR BESTIMMUNG DES FEUCHTEGEHALTS EINES FASERVERBUNDBAUTEILS**
METHOD AND MEASURING SYSTEM FOR DETERMINING THE MOISTURE CONTENT OF A FIBER COMPOSITE COMPONENT
PROCEDE ET SYSTEME DE MESURE DESTINES A DETERMINER LE TAUX D'HUMIDITE D'UN COMPOSANT EN COMPOSITE DE FIBRE

(43) Veröffentlichungstag der Anmeldung: 14.02.2018
(73) Patentinhaber: Airbus Defence and Space GmbH, 82024 Taufkirchen (DE)
(72) Erfinder: Heckner, Sebastian, 81735 München (DE); Helwig, Andreas, 80469 München (DE); Meer, Thomas, 85658 Egmating (DE); Geistbeck, Matthias, 87719 Mindelheim (DE)
(74) Vertreter: Isarpatent

(56) Entgegenhaltungen:
- DE-A1-102010 026 591
- US-A1- 2015 260 637
- US-B2- 7 807 971
- Sebastian Heckner ET AL: "FTIR SPECTROSCOPY AS A NONDESTRUCTIVE TESTING METHOD FOR CFRP SURFACES IN AEROSPACE", 7th International Symposium on NDT in Aerospace - Tu.1.A, 2015, Seiten 1-9, XP055339603, Gefunden im Internet: URL:http://www.ndt.net/article/aero2015/pa pers/tu1a3.pdf [gefunden am 2017-01-27]
- MAMDOUH AL-HARTHI ET AL: "Moisture diffusion into epoxy adhesive: testing and modeling", ADSORPTION, KLUWER ACADEMIC PUBLISHERS, BO, Bd. 13, Nr. 2, 17. Juli 2007 (2007-07-17), Seiten 115-120, XP019523026, ISSN: 1572-8757, DOI: 10.1007/S10450-007-9011-Y
- C.-H. SHEN ET AL: "Moisture Absorption and Desorption of Composite Materials", JOURNAL OF COMPOSITE MATERIALS, Bd. 10, Nr. 1, 1976, Seiten 2-20, XP055339507, USA ISSN: 0021-9983, DOI: 10.1177/002199837601000101

## Beschreibung

Die vorliegende Erfindung betrifft ein Verfahren und ein Messsystem zur Bestimmung des Feuchtegehalts eines Faserverbundbauteils.

Faserverbundbauteile finden in vielfältiger Weise Anwendung, insbesondere im Luft- und Raumfahrtbereich, im Automobilbau und im Bootsbau. Beispielsweise werden verschiedene Strukturbauteile für Rumpfstrukturen von Flugzeugen mittlerweile aus Faserverbundbauteilen hergestellt.

Üblicherweise weisen Faserverbundbauteile eine oder mehrere Schichten aus einem Fasermaterial auf, z.B. Kohlefasern, Glasfasern oder dergleichen, welche in ein Matrixmaterial, z.B. ein Epoxidharz, eingebettet sind.

Werden Faserverbundbauteile über einen gewissen Zeitraum dem Einfluss von Feuchtigkeit ausgesetzt, beispielsweise während deren Verwendung oder Transport, findet ein Transport von Feuchtigkeit in das Faserverbundbauteil hinein oder aus diesem heraus statt. Bei der Bearbeitung oder Reparatur von Faserverbundbauteilen, insbesondere wenn diese lackiert oder mit weiteren Bauteilen verklebt werden, beeinflusst der Wasser- oder Feuchtigkeitsgehalt des Faserverbundbauteils dessen mechanische Eigenschaften, z.B. die erzielbare Festigkeit einer Klebeverbindung oder die Haftfähigkeit eines Lacks an dem Faserverbundbauteil. Es besteht daher ein Bedarf zur Bestimmung des Feuchtegehalts von Faserverbundbauteilen.

Aus dem Stand der Technik sind insbesondere Verfahren bekannt, bei welchem Oberflächenkontaminierungen des Faserverbundbauteils in die Gasphase überführt und dort untersucht werden. So offenbart beispielsweise die DE 10 2011 102 055 B4 eine Vorrichtung zur Prüfung eines Faserverbundbauteils auf das Vorhandensein von Kontaminationsstoffen, wie z.B. Wasser, mit einer Oberflächenerwärmungsvorrichtung zur lokalen Erwärmung einer Bauteiloberfläche und einem Sensorarray. Durch die Erwärmung der Bauteiloberfläche werden die Kontaminationsstoffe desorbiert und in die Gasphase überführt, sodass diese von dem Sensorarray erfasst werden können.

Aus der US 7,807,971 B2 ist ein Verfahren zur Messung der Feuchtigkeit in einem Faserverbundmaterial mittels Infrarotspektroskopie, im Folgenden IR-Spektroskopie, bekannt. Hierbei werden zunächst IR-Spektren von Referenzbauteilen mit bekanntem Feuchtegehalt erstellt, mittels einem multivariaten Algorithmus ausgewertet und in einem Kalibrationsmodel dem Ergebnis der Auswertung der entsprechende Feuchtegehalt zugeordnet. Zur Bestimmung des Feuchtegehalts eines Faserverbundmaterials mit unbekanntem Feuchtegehalt wird ein IR-Spektrum dieses Faserverbundmaterials ermittelt und anhand des Kalibrationsmodels auf den Feuchtegehalt zurückgerechnet. Da IR-Strahlung in Faserverbundmaterial geringe Eindringtiefen aufweisen, wird bei Verfahren auf Basis von IR-Spektroskopie ein Feuchtegehalt des Faserverbundmaterials in Oberflächennähe zuverlässig bestimmt.

Es ist eine der Aufgaben der vorliegenden Erfindung ein Verfahren und ein Messsystem bereitzustellen, mit dem jeweils ein Gesamtfeuchtegehalt eines Faserverbundbauteils mit hoher Genauigkeit bestimmbar ist.

Diese Aufgabe wird erfindungsgemäß jeweils durch die Gegenstände der unabhängigen Ansprüche gelöst. Vorteilhafte Ausgestaltungen und Weiterbildungen ergeben sich aus den Unteransprüchen in Verbindung mit der Beschreibung.

Gemäß einem ersten Aspekt der Erfindung ist ein Verfahren zur Bestimmung des Gesamtfeuchtegehalts eines Faserverbundbauteils mit folgenden Verfahrensschritten vorgesehen:
- Aufnahme von ersten Referenzinfrarotspektren an Oberflächen von Referenzfaserverbundbauteilen, welche jeweils einen definierten Referenzfeuchtegehalt aufweisen, mittels FTIR-Messungen;
- Bilden eines spektrumsbasierten Vorhersagemodells für einen Oberflächenfeuchtegehalt durch Analyse der ersten Referenzinfrarotspektren mittels eines ersten Regressionsverfahrens;
- Ermittlung eines Referenzdiffusionsverhaltens der Referenzfaserverbundbauteile mittels mindestens einmaliger Durchführung des folgenden Ermittlungszyklus:
   - Aussetzen der Oberflächen der Referenzfaserverbundbauteile einer ersten Temperatur und einer ersten relativen Luftfeuchte über einen vorbestimmten ersten Zeitraum;
   - Aufnahme von ersten Messinfrarotspektren an den Oberflächen der Referenzfaserverbundbauteile mittels FTIR-Messungen;
   - Bestimmung von ersten Oberflächenfeuchtegehalten der Referenzfaserverbundbauteile aufgrund der ersten Messinfrarotspektren anhand des spektrumsbasiert Vorhersagemodells;
   - Aussetzen der Oberflächen der Referenzfaserverbundbauteile einer zweiten Temperatur und einer zweiten relativen Luftfeuchte über einen vorbestimmten zweiten Zeitraum;
   - Aufnahme von zweiten Messinfrarotspektren an den Oberflächen der Referenzfaserverbundbauteile mittels FTIR-Messungen; und
   - Bestimmung von zweiten Oberflächenfeuchtegehalten der Referenzfaserverbundbauteile aufgrund der zweiten Messinfrarotspektren anhand des spektrumsbasiert Vorhersagemodells;
- Bilden eines diffusionsbasierten Vorhersagemodells für den Gesamtfeuchtegehalt durch Analyse des Referenzdiffusionsverhaltens mittels eines zweiten Regressionsverfahrens;
- Ermittlung eines tatsächlichen Diffusionsverhaltens des Faserverbundbauteils mittels mindestens einmaliger Durchführung des Ermittlungszyklus an dem Faserverbundbauteil; und
- Bestimmung des tatsächlichen Gesamtfeuchtegehalts des Faserverbundbauteils aufgrund des tatsächlichen Diffusionsverhaltens des Faserverbundbauteils anhand des diffusionsbasierten Vorhersagemodells.

Erfindungsgemäß erfolgt zunächst das Erstellen eines spektrumsbasierten Vorhersagemodells für die Feuchtigkeit eines Faserverbundbauteils. Hierzu werden Referenzinfrarotspektren von Referenzfaserverbundbauteilen aufgenommen. Die Referenzfaserverbundbauteile weisen einen definierten, bekannten Feuchtegehalt auf und sind homogen mit Feuchtigkeit gesättigt. Für jeden Feuchtegehalt wird ein Referenzinfrarotspektrum gebildet. Die Referenzinfrarotspektren werden mittels einer Fourier-Transform-Infrarot-Messung, im Folgenden als FTIR-Messung bezeichnet, durchgeführt. Die den jeweiligen bekannten Feuchtegehalten zugeordneten Referenzinfrarotspektren werden mittels eines Regressionsverfahrens analysiert. Auf diese Weise wird ein funktionaler Zusammenhang zwischen bestimmten Charakteristika der Referenzinfrarotspektren und dem Feuchtegehalt ermittelt und somit ein spektrumsbasiertes Vorhersagemodells für den Feuchtegehalt gebildet. Aufgrund der geringen Eindringtiefe von IR-Strahlung in das Faserverbundbauteil wird hierbei insbesondere ein Modell für die Vorhersage der an der Oberfläche des Referenzbauteils vorliegenden Feuchte bereitgestellt. Dies ermöglicht in einfacher Weise die schnelle und genaue Bestimmung der Oberflächenfeuchte der Referenzbauteile, was im weiteren Verfahren vorteilhaft genutzt wird.

Erfindungsgemäß wird nach dem Bilden des spektrumsbasierten Vorhersagemodells für den Feuchtegehalt ein Ermittlungszyklus zur Ermittlung eines Referenzdiffusionsverhaltens der Referenzfaserverbundbauteile durchgeführt. Der Ermittlungszyklus umfasst eine bestimmte Sequenz von Wärmebehandlungen und FTIR-Messungen. Zunächst wird die Oberfläche des jeweiligen Referenzbauteils über einen vorbestimmten ersten Zeitraum mit einer bestimmten ersten Temperatur und einer ersten relativen Luftfeuchte beaufschlagt. Die erste Temperatur, die erste relative Luftfeuchte und der erste Zeitraum sind für alle Referenzbauteile gleich. Auf diese Weise wird ein Diffusionsvorgang von Feuchtigkeit in das Referenzbauteil hinein oder aus diesem heraus bewirkt, wodurch für jedes Referenzbauteil eine eigene Startbedingung für die oberflächliche Feuchtigkeitssättigung eingestellt wird. Anschließend wird an der Oberfläche des jeweiligen Referenzfaserverbundbauteils mittels FTIR-Messungen ein erstes Messinfrarotspektrum aufgenommen. Aus diesem wird weiterhin ein erster Oberflächenfeuchtegehalt des Referenzfaserverbundbauteils bestimmt. Hierzu wird das spektrumsbasierte Vorhersagemodell für den Feuchtegehalt, in welchem ein Feuchtegehalt als Funktion der Charakteristika eines IR-Spektrums vorliegt, genutzt. Weiterhin wird die jeweilige Oberfläche des jeweiligen Referenzbauteils über einen vorbestimmten zweiten Zeitraum einer zweiten Temperatur und einer zweiten relativen Luftfeuchte ausgesetzt. Damit erfolgt ein weiterer Diffusionsvorgang von Feuchtigkeit in das Referenzbauteil hinein oder aus diesem heraus, welcher eine Änderung der Feuchtigkeitssättigung des Referenzfaserverbundbauteils bewirkt. Diese Änderung ist abhängig vom Gesamtfeuchtegehalt des Referenzfaserverbundbauteils. Es wird ein zweites Messinfrarotspektrum an der Oberfläche des jeweiligen Referenzfaserverbundbauteils mittels FTIR-Messung aufgenommen und ein geänderter, zweiter Oberflächenfeuchtegehalt der Referenzfaserverbundbauteile mittels des spektrumsbasierten Vorhersagemodells bestimmt. Die Änderung des Oberflächenfeuchtegehalts während der jeweiligen Temperatur- bzw. Feuchtigkeitsbeaufschlagung stellt das Diffusionsverhalten dar, welche abhängig vom Gesamtfeuchtegehalt des Referenzfaserverbundbauteils ist. Der Ermittlungszyklus wird einmal oder mehrmals nacheinander durchgeführt.

Die auf diese Weise ermittelten, dem jeweiligen bekannten Feuchtegehalt des jeweiligen Referenzbauteils zugeordneten Referenzdiffusionsverhalten werden mittels eines Regressionsverfahrens analysiert. Auf diese Weise wird ein funktionaler Zusammenhang zwischen bestimmten Charakteristika des Diffusionsverhaltens und dem Feuchtegehalt ermittelt und somit ein diffusionsbasiertes Vorhersagemodells für den Feuchtegehalt gebildet.

Zur Bestimmung des Feuchtegehalts des Faserverbundbauteils mit unbekanntem Feuchtegehalt wird zunächst dessen tatsächliches Diffusionsverhalten mittels mindestens einmaliger Durchführung des Ermittlungszyklus an dem Faserverbundbauteil bestimmt. Hierbei werden exakt die gleichen Schritte, welche an den Referenzbauteilen durchgeführt wurden, auch an dem Faserverbundbauteil durchgeführt. Der Ermittlungszyklus bietet den Vorteil, dass die Oberfläche des Faserverbundbauteils zunächst über den vorbestimmten ersten Zeitraum mit der bestimmten ersten Temperatur und der ersten relativen Luftfeuchte beaufschlagt wird. Damit wird eine vorhandene Oberflächenfeuchte des Faserverbundbauteils gewissermaßen "gelöscht" und eine Startbedingung zur Ermittlung des von der Gesamtbauteilfeuchte abhängigen Diffusionsverhaltens eingestellt. Bei der Beaufschlagung der Oberfläche des Faserverbundbauteils über den zweiten Zeitraum mit der zweiten Temperatur und der zweiten relativen Luftfeuchte erfolgt eine Änderung der Oberflächenfeuchte des Faserverbundbauteils. Dieser Feuchteaustausch wird insbesondere von einem Gradienten des Feuchtigkeitsgehalts zwischen einem oberflächennahen Dickenbereich des Faserverbundbauteils und einem Kernbereich des Faserverbundbauteils beeinflusst. Die Bestimmung des Gesamtfeuchtegehalts des Faserverbundbauteils erfolgt anhand des diffusionsbasierten Vorhersagemodells. Hierbei bildet das aus dem Ermittlungszyklus ermittelte tatsächliche Diffusionsverhalten des Faserverbundbauteils die in den durch das diffusionsbasierte Vorhersagemodell bereitgestellten funktionalen Zusammenhang einzusetzende Variable.

Das erfindungsgemäße Verfahren bietet den Vorteil, dass mittels des diffusionsbasierten Vorhersagemodells letztlich aus einer an der Oberfläche eines Faserverbundbauteils gemessenen Feuchte auf einfache und zuverlässige Weise auf die Gesamtfeuchte des Bauteils geschlossen werden kann. Damit kann ein schnell und mit hoher Genauigkeit durchführbares FTIR-Messverfahren zur Bestimmung des Feuchtegehalts des Faserverbundbauteils insgesamt genutzt werden. Durch den Ermittlungszyklus ist ein Diffusionsverhalten des Faserverbundbauteils und der Referenzfaserverbundbauteile in vorteilhafter und einfacher Weise ermittelbar.

Die Referenzfaserverbundbauteile, im Folgenden aus Gründen der sprachlichen Vereinfachung auch Referenzbauteile genannt, weisen jeweils unterschiedliche Gesamtfeuchtegehalte auf. Mehrere Referenzbauteile können hierbei insbesondere durch ein einziges Bauteil bereitgestellt werden, dessen Feuchtegehalt nach der Durchführung bestimmter Verfahrensschritte, insbesondere nach Durchführung der Aufnahme der ersten Referenzinfrarotspektren und/oder des Ermittlungszyklus verändert wird. Selbstverständlich können auch mehrere verschiedene Bauteile, welche jeweils zueinander unterschiedliche Feuchtegehalte aufweisen, als Referenzbauteile verwendet werden.

Innerhalb der Referenzfaserverbundbauteile liegt bis zu Beginn des Ermittlungszyklus eine über eine Dickenrichtung der Bauteile homogene Feuchtigkeitsverteilung vor. Weiterhin weisen die Referenzfaserverbundbauteile dieselbe Materialzusammensetzung, insbesondere gleiche Matrixmaterialien und Fasermaterialien sowie gleichen Faser- bzw. Matrixmaterialanteile, auf.

Ein Referenzinfrarotspektrum kann insbesondere dadurch aufgenommen werden, dass an der Oberfläche eines Referenzbauteils mehrere, insbesondere zumindest zwei, bevorzugt zumindest 10 Einzelinfrarotspektren desselben Oberflächenbereichs durch FTIR-Messung ermittelt und aus diesen ein das Referenzinfrarotspektrum bildender Mittelwert berechnet wird. Dies bietet den Vorteil, dass die Genauigkeit des aufgrund des Referenzinfrarotspektrums gebildeten spektrumsbasierten Vorhersagemodells verbessert wird, da der Einfluss von Messfehlern durch die Mittelwertbildung verringert wird.

Gemäß einer vorteilhaften Ausführungsform ist vorgesehen, dass das erste Regressionsverfahren eine multivariate, d.h. mehrdimensionale, Analyse des jeweiligen Referenzinfrarotspektrums umfasst. In dem Verfahren gemäß dieser Weiterbildung können somit bekannte mathematische bzw. statistische Werkzeuge aus dem Bereich der multivariaten Datenanalyse Verwendung finden. Durch die Nutzung derartiger Analyseverfahren können die für den Feuchtegehalt maßgeblichen Charakteristika oder Parameter des Referenzspektrums mit hoher Genauigkeit und Zuverlässigkeit bestimmt werden, da hier mehrere Variablen in die Analyse einfließen. Der Fachmann wird entsprechend zwischen verschiedenen Analysemethoden wählen können, um einen für die jeweilige Anwendung geeigneten Kompromiss zwischen Genauigkeit und Komplexität, d.h. letztendlich der Zeitdauer, der Analyse zu finden.

Ein für den Feuchtegehalt maßgebliches Charakteristikum oder ein maßgeblicher Parameter des Referenzspektrums kann insbesondere durch die Absorptionsintensität in den Absorptionsbandenlagen von Wasser im FTIR-Spektrum gegeben sein.

Gemäß einer vorteilhaften Weiterbildung umfasst das zweite Regressionsverfahren eine multivariate Analyse des Referenzdiffusionsverhaltens. Die obigen Ausführungen zu den Vorteilen der multivariaten Analyse des ersten Regressionsverfahrens gelten entsprechend. Als das das Referenzdiffusionsverhalten kennzeichnender Parameter kann insbesondere die betragsmäßige Änderung des Oberflächenfeuchtegehalts während einer einmaligen Durchführung des Ermittlungszyklus und/oder der Verlauf der Änderung über mehrere Ermittlungszyklen herangezogen werden.

Gemäß einer bevorzugten Ausführungsform umfasst die multivariate Analyse die Durchführung eines PLS-Algorithmus. "PLS" steht hierbei als Abkürzung für den englischen Ausdruck "Partial Least Squares". Dieser an sich bekannte Algorithmus bietet den Vorteil, dass mit relativ geringem Rechenaufwand Ergebnisse von hoher Genauigkeit erzielt werden.

Eine weitere vorteilhafte Ausführungsform des Verfahrens sieht vor, dass bei dem Ermittlungszyklus nach der Bestimmung der ersten Oberflächenfeuchtegehalte der Referenzfaserverbundbauteile die Schritte des Aussetzens der Oberflächen der Referenzfaserverbundbauteile der ersten Temperatur und der ersten relativen Luftfeuchte, der Aufnahme der ersten Messinfrarotspektren und der Bestimmung der ersten Oberflächenfeuchtegehalte der Referenzfaserverbundbauteile wiederholt werden. Demnach erfolgt nach der Bestimmung der ersten Oberflächenfeuchte ein erneutes Aussetzten der Oberflächen der Referenzfaserverbundbauteile der ersten Temperatur und der ersten relativen Luftfeuchte über einen vorbestimmten Zeitraum, insbesondere den ersten Zeitraum. Dadurch wird vorteilhaft eine Zuverlässige Einstellung der Oberflächenfeuchte sichergestellt. Anschließend erfolgt nochmals die Aufnahme von Messinfrarotspektren und die Bestimmung der ersten Oberflächenfeuchtegehalte. Dieses Vorgehen bietet zusätzlich den Vorteil, dass ein weiterer Diffusionsvorgang innerhalb des Referenzbauteils oder des Faserverbundbauteils bewirkt wird, wodurch das Diffusionsverhalten genauer bestimmbar ist.

Die Referenzfeuchtegehalte der Referenzfaserverbundbauteile liegen bevorzugt zwischen Null Gewichtsprozent und 3 Gewichtsprozent. Auf diese Weise wird sichergestellt, dass der üblicherweise an Faserverbundbauteilen auftretende tatsächliche Feuchtegehalt zuverlässig von dem spektrumsbasierten Vorhersagemodell und dem diffusionsbasierten Vorhersagemodell umfasst ist.

Insbesondere kann vorgesehen sein, dass die Referenzfeuchtegehalte der verschiedenen Referenzfaserverbundbauteile sich jeweils zwischen 0,01 und 0,2 Gewichtsprozent unterscheiden. In diesem Bereich der Schrittweite der Änderung des Referenzfeuchtegehalts wird eine gute Auflösung für die Bestimmung des tatsächlichen Feuchtegehalts des Faserverbundbauteils gewährleistet.

Gemäß einer vorteilhaften Weiterbildung des Verfahrens liegt die erste Temperatur in einem Bereich zwischen 90 Grad Celsius und 200 Grad Celsius und die erste relative Luftfeuchte der ersten in einem Bereich zwischen 0 Prozent und 50 Prozent. In diesem Temperaturbereich laufen die Diffusionsvorgänge mit einer Geschwindigkeit ab, welche eine zügige Durchführung des Ermittlungszyklus ermöglicht, ohne das Referenzbauteil oder das Faserverbundbauteil zu beschädigen.

Der erste Zeitraum kann insbesondere zwischen 0,5 Minuten und 30 Minuten betragen. Innerhalb dieser Zeitspanne werden Änderungen der Oberflächenfeuchten erzielt, welche groß genug sind um eine ursprünglich vorhandene Oberflächenfeuchte des Faserverbundbauteils gewissermaßen zu "löschen" und eine Startbedingung zur Ermittlung des von der Gesamtbauteilfeuchte abhängigen Diffusionsverhaltens bereitzustellen.

Gemäß einer besonders vorteilhaften Weiterbildung liegt die zweite Temperatur in einem Bereich zwischen 40 Grad Celsius und 200 Grad Celsius und die zweite relative Luftfeuchte in einem Bereich zwischen größer 50 Prozent und kleiner oder gleich 100 Prozent. Eine hohe Temperatur bewirkt hierbei vorteilhaft einen schnellen Ablauf der Diffusion. Durch die gegenüber der ersten relativen Luftfeuchte erhöhten zweiten relativen Luftfeuchte wird in der Regel ein Wiederanfeuchten der Oberfläche der Bauteile bewirkt. Die Änderung der Oberflächenfeuchte ist abhängig vom Gesamtfeuchtigkeitsgehalt des Bauteils. In dem vorgenannten Bereich werden vorteilhaft für Bauteile verschiedener Gesamtfeuchte deutlich unterschiedlich große Änderungen der Oberflächenfeuchte erzielt. In diesem Bereich der relativen Luftfeuchte sind die Diffusionsverhalten für verschiedene Gesamtfeuchten somit besonders gut unterscheidbar, sodass das Regressionsverfahren ein sehr genaues Referenzdiffusionsmodell liefert.

Bevorzugt beträgt der zweite Zeitraum zwischen 0,5 Minuten und 24 Stunden. Ein kurzer zweiter Zeitraum bietet den Vorteil einer schnellen Durchführbarkeit des Verfahrens. Ein langer zweiter Zeitraum bietet den Vorteil einer besonders deutlichen Änderung der Oberflächenfeuchte.

Gemäß einer weiteren vorteilhaften Ausführungsform des Verfahrens ist vorgesehen, dass die erste und die zweite Temperatur und die erste und die zweite relative Luftfeuchte jeweils in einem Arbeitsraum erzeugt werden, welcher durch eine an der Oberfläche des Referenzfaserverbundbauteils oder an der Oberfläche des Faserverbundbauteils anliegende Glocke begrenzt wird. Demgemäß wird eine Glocke, welche eine eine Öffnung aufweisende Kavität aufweist, derart an die Oberfläche des Bauteils angelegt, dass die Oberfläche des Bauteils die Öffnung der Kavität abdeckt. Dadurch begrenzen die Glocke und die Oberfläche den Arbeitsraum. Auf diese Weise wird vorteilhaft ein geschlossener Raum ausgebildet, in welchem die thermodynamischen Bedingungen, insbesondere die Temperatur und die relative Luftfeuchte, auf einfache Weise kontrollierbar und einstellbar sind. Darüber hinaus ist es durch die Glocke möglich, lediglich einen Teilbereich der Oberfläche des Referenzfaserverbundbauteils oder des Faserverbundbauteils den gewünschten thermodynamischen Bedingungen auszusetzten. Dadurch wird sowohl der Energieaufwand als auch der Platzbedarf zur Durchführung des Verfahrens verringert.

Eine besonders vorteilhafte Weiterbildung des Verfahrens sieht vor, dass die Glocke zur Aufnahme der ersten Messinfrarotspektren und zur Aufnahme der zweiten Messinfrarotspektren von der Oberfläche des Referenzfaserverbundbauteils oder an der Oberfläche des Faserverbundbauteils entfernt wird. Demgemäß wird während des Ermittlungszyklus jeweils die erste bzw. zweite Temperatur bei der jeweiligen ersten bzw. zweiten relativen Luftfeuchtigkeit unter der Glocke eingestellt und über den jeweiligen ersten bzw. zweiten Zeitraum gehalten. Nach Ablauf des ersten bzw. zweiten Zeitraums wird die Glocke entfernt und das jeweilige Infrarotspektrum in dem Oberflächenbereich, welcher durch die Glocke bedeckt war, ermittelt. Dies bietet den Vorteil, dass jeweils eine einfach aufgebaute Glocke und eine einfach aufgebaute Vorrichtung zur Ermittlung der Infrarotspektren, beispielsweise in Form eines FTIR-Spektrometers, verwendet werden kann. Insbesondere wird die Verwendung von tragbaren Geräten ermöglicht.

Nach einem zweiten Aspekt der Erfindung ist ein nicht-flüchtigen Datenspeicher vorgesehen, welcher ein Programm speichert, das dazu ausgelegt ist, einen Computer zur Ausführung eines Verfahrens nach einer der voranstehend beschriebenen Ausführungsformen zu veranlassen. Der nicht-flüchtige Datenspeicher kann insbesondere als eine Festplatte, eine CD-ROM, eine Diskette, einen Flash-Speicher oder dergleichen ausgebildet sein.

Nach einem zweiten Aspekt der Erfindung ist ein Messsystem zur Messung der Feuchtigkeit des Faserverbundbauteils vorgesehen. Diese weist eine Glocke zur Einstellung der ersten Temperatur und einer ersten relativen Luftfeuchtigkeit sowie einer zweiten Temperatur und einer zweiten relativen Luftfeuchtigkeit an einer Oberfläche des Faserverbundbauteils, ein Spektrometer, einen Computer, insbesondere mit einer Auswerteeinheit, an welchen die Glocke und das Spektrometer funktional gekoppelt sind, und einen von dem Computer lesbaren, nicht-flüchtiger Datenspeicher gemäß der voranstehenden Beschreibung auf. Unter einem "Feuchtegehalt", einem "Gesamtfeuchtegehalt", "einer Gesamtfeuchte" oder "einer Feuchtigkeitssättigung" eines Bauteils, insbesondere des Faserverbundbauteils oder des Referenzbauteils, wird hierin allgemein ein auf das Gesamtgewicht des feuchten Bauteils bezogenes Gewicht der in dem Bauteil vorhandenen Feuchtigkeit verstanden.

Unter einem "Oberflächenfeuchtegehalt" eines Bauteils wird hierin eine angenommene Gesamtfeuchte des Bauteils verstanden, welche aus einem an der Oberfläche des Bauteils aufgenommenen Infrarotspektrum ermittelt wurde.

Im Folgenden wird die Erfindung unter Bezugnahme auf die Figuren der Zeichnungen erläutert. Von den Figuren zeigen:
- Fig. 1: ein schematisches Ablaufdiagramm eines Verfahrens gemäß einem bevorzugten Ausführungsbeispiel der vorliegenden Erfindung;
- Fig. 2: eine schematische Darstellung von in einem ersten Schritt des Verfahrens ermittelten Referenzinfrarotspektren;
- Fig. 3: eine schematische Darstellung eines Ermittlungszyklus gemäß dem bevorzugten Ausführungsbeispiel des Verfahrens der vorliegenden Erfindung;
- Fig. 4: eine schematische Darstellung erster Messinfrarotspektren, welche während des Ermittlungszyklus des Verfahrens ermittelt wurden;
- Fig. 5: eine schematische Darstellung von durch den Ermittlungszyklus des Verfahrens ermittelten Referenzdiffusionsverhalten von Referenzfaserverbundbauteilen;
- Fig. 6: eine schematische Darstellung eines durch den Ermittlungszyklus des Verfahrens ermittelten tatsächlichen Diffusionsverhaltens eines Faserverbundbauteils;
- Fig. 7: eine schematische Darstellung der Durchführung eines Schritts des Ermittlungszyklus des Verfahrens gemäß einem Ausführungsbeispiel der vorliegenden Erfindung;
- Fig. 8: eine schematische Darstellung der Durchführung eines Schritts des der Ermittlung eines Infrarotspektrums gemäß einem Ausführungsbeispiel des Verfahrens nach der vorliegenden Erfindung; und
- Fig. 9: eine schematische Darstellung eines Messsystems gemäß einem Ausführungsbeispiel der vorliegenden Erfindung.

In den Figuren bezeichnen dieselben Bezugszeichen gleiche oder funktionsgleiche Komponenten, soweit nichts Gegenteiliges angegeben ist.

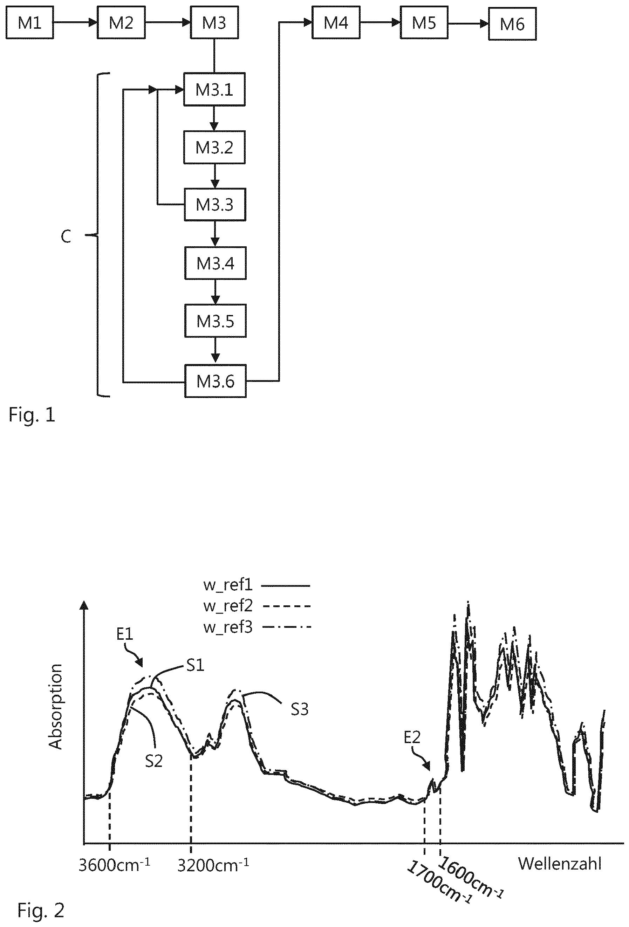

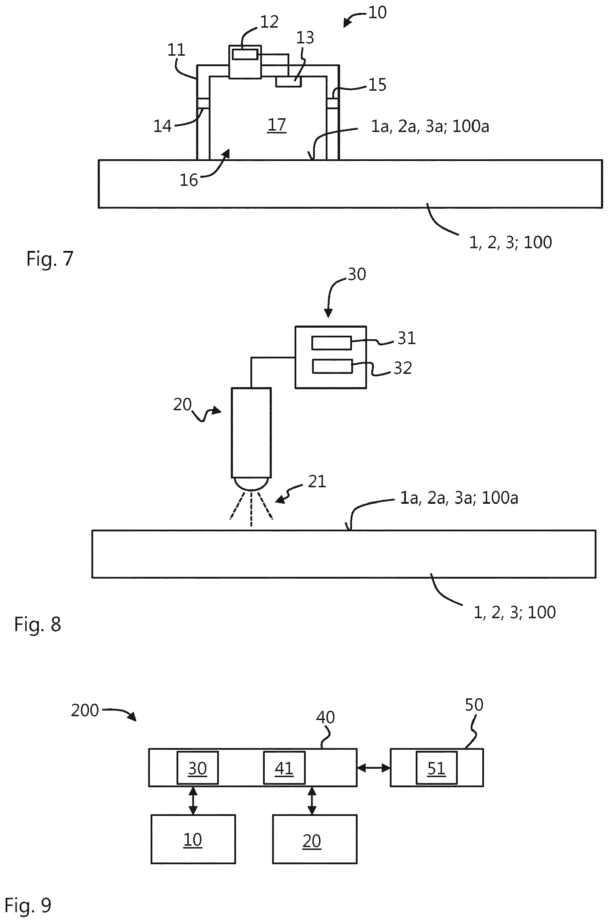

Fig. 1 zeigt schematisch den Ablauf eines Verfahrens M zur Bestimmung des Feuchtegehalts eines Faserverbundbauteils 100. In einem ersten Verfahrensschritt M1 erfolgt eine Aufnahme von ersten Referenzinfrarotspektren S1, S2, S3 an Oberflächen 1a, 2a, 3a von Referenzfaserverbundbauteilen 1, 2, 3. Die Referenzfaserverbundbauteilen 1, 2, 3 weisen jeweils einen definierten, bekannten Referenzfeuchtegehalt w_ref1, w_ref2, w_ref3 und homogene räumliche Feuchteverteilung auf. Die Referenzinfrarotspektren S1, S2, S3 werden jeweils mittels FTIR-Messung, also mittels einer Fourier-Transform-Infrarot-Messung, aufgenommen. Hierzu kann insbesondere ein FTIR-Spektrometer 20 verwendet werden, wie in Fig. 8 beispielhaft und schematisch dargestellt ist. Mit dem FTIR-Spektrometer 20 wird IR-Strahlung 21 aus einem bestimmten Wellenlängenbereich auf die Oberfläche 1a, 2a, 3a des jeweiligen Referenzfaserverbundbauteils 1, 2, 3 gerichtet und mittels eines Detektors (nicht gezeigt) die Absorption der IR-Strahlung an der Oberfläche 1a, 2a, 3a des jeweiligen Referenzfaserverbundbauteils 1, 2, 3 in Abhängigkeit der Wellenlänge erfasst.

Das Spektrometer 20 kann funktional an Analysevorrichtung 30 gekoppelt sein, welche eine Datenverarbeitungseinrichtung 31, insbesondere in Form eines Prozessors, und einen Datenspeicher 32 aufweist. Die Referenzinfrarotspektren S1, S2, S3 können als Daten insbesondere in dem Datenspeicher 32 abgelegt werden. Auch kann durch die Datenverarbeitungseinrichtung 31 eine Bearbeitung der Daten der Referenzinfrarotspektren S1, S2, S3 erfolgen, beispielsweise eine Mittelung, falls für jedes Referenzbauteil 1, 2, 3 mehrere Infrarotspektren aufgenommen werden.

Fig. 2 zeigt beispielhaft und schematisch ein erstes Referenzinfrarotspektrum S1 eines ersten Referenzfaserverbundbauteils 1, welches einen ersten Referenzfeuchtegehalt w_ref1 aufweist, ein erstes Referenzinfrarotspektrum S2 eines zweiten Referenzfaserverbundbauteils 2, welches einen zweiten Referenzfeuchtegehalt w_ref2 aufweist, und ein erstes Referenzinfrarotspektrum S3 eines dritten Referenzfaserverbundbauteils 3, welches einen dritten Referenzfeuchtegehalt w_ref3 aufweist. Bei den in Fig. 2 dargestellten Referenzinfrarotspektren S1, S2, S3 ist die Absorption der IR-Strahlung an der jeweiligen Oberfläche 1a, 2a, 3a des jeweiligen Referenzfaserverbundbauteils 1, 2, 3 auf der Ordinate und eine Wellenzahl, also der Kehrwert der Wellenlänge der IR-Strahlung, auf der Abszisse aufgetragen. Die Wellenzahl weist hierbei an der Schnittstelle Abszisse mit der Ordinate einen Maximalwert auf und nimmt in mathematisch positiver Richtung entlang der Abszisse ab. Die entspricht einer Zunahme der Wellenlänge. Die Referenzinfrarotspektren S1, S2, S3 weisen einen qualitativ ähnlichen Verlauf auf. Insbesondere bildet die Absorption in einem Wellenzahlbereich zwischen etwa 3600 cm⁻¹ und etwa 3200 cm⁻¹ ein Maximum E1 aus. Dieses Absorptionsmaximum E1 resultiert aus Schwingungen von OH-Molekülen in den Referenzfaserverbundbauteilen 1, 2, 3 und zeigen daher das Vorhandensein eines bestimmten Feuchtegehalts in den Referenzfaserverbundbauteilen 1, 2, 3 an. Weiterhin bildet die Absorption in einem Wellenzahlbereich zwischen etwa 1600 cm⁻¹ und etwa 1700 cm⁻¹ ein weiteres Absorptionsmaximum E2 aus. Dieses Maximum E2 resultiert aus Schwingungen von HOH-Molekülen in den Referenzfaserverbundbauteilen 1, 2, 3 und zeigt daher ebenfalls das Vorhandensein eines bestimmten Feuchtegehalts in den Referenzfaserverbundbauteilen 1, 2, 3 an. Da die Referenzfeuchtegehalte w_ref1, w_ref2, w_ref3 der Referenzfaserverbundbauteile 1, 2, 3 jeweils zueinander verschieden sind, unterscheiden sich die Verläufe der Referenzinfrarotspektren S1, S2, S3 insbesondere im Bereich der genannten Absorptionsmaxima E1, E2.

In einem zweiten Schritt M2 des Verfahrens wird ein spektrumsbasiertes Vorhersagemodell für den Feuchtegehalt gebildet. Dies erfolgt durch Analyse der ersten Referenzinfrarotspektren S1, S2, S3 mittels eines ersten Regressionsverfahrens. Hierbei wird beispielsweise der typische wellenzahlabhängige Verlauf der Absorption in dem Wellenzahlbereich, in welchem die Absorptionsmaxima E1, E2 liegen, für den jeweiligen Referenzfeuchtegehalt w_ref1, w_ref2, w_ref3 quantitativ analysiert und dem Verlauf der jeweilige Referenzfeuchtegehalt w_ref1, w_ref2, w_ref3 zugeordnet. Das spektrumsbasierte Vorhersagemodell gibt somit den Referenzfeuchtegehalt w_ref1, w_ref2, w_ref3 als Funktion von Parametern der Referenzspektren S1, S2, S3 an.

Die Analyse der Referenzinfrarotspektren S1, S2, S3 kann insbesondere in der funktional mit dem Spektrometer 20 gekoppelten Analysevorrichtung 30 erfolgen. Das spektrumsbasierte Vorhersagemodell für den Feuchtegehalt kann ebenfalls mit Hilfe der Datenverarbeitungseinrichtung 31 erstellt und in dem Datenspeicher 32 der Analysevorrichtung 30 abgelegt werden. In Fig. 8 ist die Analysevorrichtung 30 schematisch dargestellt.

In einem weiteren Verfahrensschritt M3 wird ein Referenzdiffusionsverhalten D_refl, D_ref2, D_ref3 der Referenzfaserverbundbauteile 1, 2, 3 ermittelt. Hierzu wird mindestens einmal ein Ermittlungszyklus C durchgeführt. Die Schritte M3.1 bis M3.6 des Ermittlungszyklus C sind in Fig. 1 schematisch als Ablaufdiagramm dargestellt.

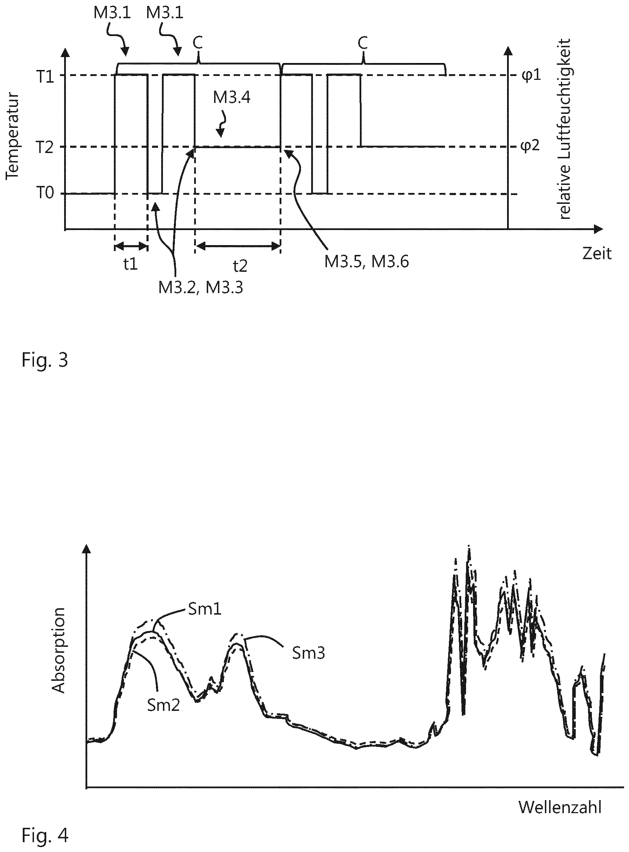

Fig. 3 zeigt schematisch den Verlauf des Ermittlungszyklus C als Verlauf einer Temperatur an der Oberfläche 1a, 2a, 3a des jeweiligen Referenzbauteils 1, 2, 3 abhängig von der Zeit. In einem ersten Schritt M3.1 des Ermittlungszyklus C erfolgt eine erste Wärmebehandlung der Referenzbauteile 1, 2, 4. Hierbei werden die Oberflächen 1a, 2a, 3a der Referenzfaserverbundbauteile 1, 2, 3 über einen vorbestimmten ersten Zeitraum t1 einer ersten Temperatur T1 und einer ersten relativen Luftfeuchte ϕ1 ausgesetzt, wobei die erste Temperatur T1 und die erste relative Luftfeuchte ϕ1 vorzugsweise konstant sind, wie in Fig. 3 schematisch gezeigt ist. Die erste Temperatur T1 kann insbesondere in einem Bereich zwischen 90 Grad Celsius und 200 Grad Celsius und die erste relative Luftfeuchte ϕ1 der ersten in einem Bereich zwischen 0 Prozent und 50Prozent liegen. Beispielsweise beträgt die erste Temperatur T1 bevorzugt 180 Grad Celsius und die erste relative Luftfeuchte ϕ1 liegt nach 0 Prozent, beispielsweise bei 0,5 Prozent. Bei dem in Fig. 3 gezeigten Ausführungsbeispiel werden die Referenzbauteile von einer Ausgangstemperatur T0 auf die erste Temperatur T1 erwärmt anschließend wieder auf die Ausgangstemperatur T0 abgekühlt.

In einem zweiten Schritt M3.2 des Ermittlungszyklus C erfolgt eine Aufnahme von ersten Messinfrarotspektren Sm1, Sm2, Sm3 an den Oberflächen 1a, 2a, 3a der Referenzfaserverbundbauteile 1, 2, 3 mittels FTIR-Messungen, wie oben für den ersten Verfahrensschritt M1 anhand Fig. 8 beschrieben. Fig. 4 zeigt beispielhaft und schematisch ein erstes Messinfrarotspektrum Sm1 des ersten Referenzfaserverbundbauteils 1, ein erstes Messinfrarotspektrum Sm2 des zweiten Referenzfaserverbundbauteils 2 und ein erstes Messinfrarotspektrum Sm1 des dritten Referenzfaserverbundbauteils 3. Diese ersten Messinfrarotspektren Sm1, Sm2, Sm3 sind qualitativ ähnlich zu den ersten Referenzinfrarotspektren S1, S2, S3, wie Fig. 4 zeigt.

In einem dritten Schritt M3.3 des Ermittlungszyklus C werden erste Oberflächenfeuchtegehalte der Referenzfaserverbundbauteile 1, 2, 3 aus den ersten Messinfrarotspektren Sm1, Sm2, Sm3 bestimmt. Hierzu wird das spektrumsbasierte Vorhersagemodell für den Feuchtegehalt genutzt. Insbesondere kann die Bestimmung mit dem spektrumsbasierten Vorhersagemodell eine Auswertung der den Feuchtigkeitsgehalt kennzeichnenden Parameter der ersten Messinfrarotspektren Sm1, Sm2, Sm3 umfassen. Diese Parameter werden dann als Eingangsgröße für den durch das spektrumsbasierte Vorhersagemodell gegebenen funktionalen Zusammenhang zwischen den Parametern der Referenzinfrarotspektren S1, S2, S3 und dem Feuchtegehalt verwendet und auf diese Weise der Oberflächenfeuchtegehalt der Referenzbauteile 1, 2, 3 bestimmt, welcher nach der ersten Wärmebehandlung vorliegt.

Die Bestimmung des Feuchtegehalts mit Hilfe des spektrumsbasierten Vorhersagemodells kann ebenfalls durch die Datenverarbeitungseinrichtung 31 der Analysevorrichtung 30 erfolgen. Hierzu werden die Daten der mittels dem Spektrometer 20 aufgenommenen ersten Messinfrarotspektren Sm1, Sm2, Sm3 in der Datenverarbeitungseinrichtung 31 hinsichtlich bestimmter Parameter analysiert und das Ergebnis dieser Analyse als Input für das im Datenspeicher 32 abgelegte spektrumsbasierte Vorhersagemodell verwendet. Der ermittelte Oberflächenfeuchtegehalt kann in dem Datenspeicher 32 der Analysevorrichtung 30 abgelegt werden.

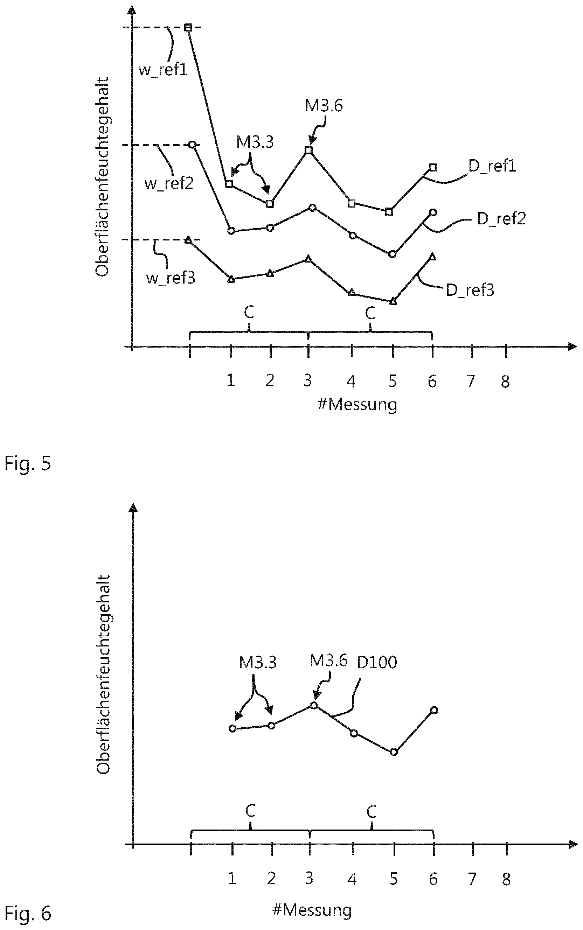

Fig. 5 zeigt schematisch das durch zweimalige Durchführung des Ermittlungszyklus C ermittelte Referenzdiffusionsverhalten D_ref1, D_ref2. D_ref3 der Referenzbauteile 1, 2, 3. Auf der Ordinate ist hierbei der aufgrund der FTIR-Messung ermittelte Oberflächenfeuchtegehalt der Referenzbauteile 1, 2, 3 aufgetragen. Entlang der Abszisse ist eine Ordnungsnummer der jeweiligen FTIR-Messung innerhalb des Ermittlungszyklus C aufgetragen. Wie Fig. 5 zeigt, liegen die Referenzbauteile 1, 2, 3 vor Beginn des Ermittlungszyklus C jeweils mit ihrer Referenzfeuchte w_ref1, w_ref2, w_ref3 vor, wobei die Referenzfeuchte w_ref1 des ersten Referenzbauteils 1 größer ist als die Referenzfeuchten w_ref2, w_ref3 des zweiten und des dritten Referenzbauteils 2, 3. Die die Referenzfeuchte w_ref3 des dritten Referenzbauteils 3 ist kleiner als die Referenzfeuchten w_ref1, w_ref2 des ersten und des zweiten Referenzbauteils 1, 2. Die Referenzfeuchten w_ref1, w_ref2, w_ref3 sind für die Referenzbauteile 1, 2, 3 bekannt und können beispielsweise durch ein Wiegeverfahren ermittelt worden sein, bei dem das Gewicht der trockenen Referenzbauteile 1, 2, 3 sowie das Gewicht der befeuchteten Referenzbauteile 1, 2, 3 ermittelt und durch den Unterschied der ermittelten Gewichte die Referenzfeuchten w_ref1, w_ref2, w_ref3 bestimmt wurden. Fig. 5 zeigt schematisch, dass bei den vorliegenden Referenzfeuchten w_ref1, w_ref2, w_ref3 und der gewählten ersten Temperatur T1 und der gewählten ersten relativen Luftfeuchtigkeit ϕ1 der aus der FTIR-Messung #1, welcher dem Schritt M3.3 des Ermittlungszyklus entspricht, ermittelte Oberflächenfeuchtegehalt nach der ersten Wärmebehandlung deutlich kleiner ist als der jeweilige Referenzfeuchtegehalt w_ref1, w_ref2, w_ref3.

Wie in Fig. 1 schematisch zeigt, kann die erste Wärmebehandlung mit den ersten drei Schritten M3.1, M3.2, M3.3 des Ermittlungszyklus C optional zumindest einmal wiederholt werden. Hierbei wird vorzugsweise dieselbe erste Temperatur T1 und dieselbe erste relative Luftfeuchtigkeit ϕ1 eingestellt, wie beispielhaft in Fig. 3 gezeigt ist. Fig. 5 zeigt beispielhaft, dass diese Wiederholung der ersten Wärmebehandlung für das zweite und das dritte Referenzbauteil 2, 3 jeweils einen leichten Anstieg des aus der FTIR-Messung #2, welcher wiederum dem Schritt M3.3 des Ermittlungszyklus entspricht, ermittelten Oberflächenfeuchtegehalt bewirkt.

In einem weiteren Schritt M3.4 des Ermittlungszyklus C werden die Oberflächen 1a, 2a, 3a der Referenzfaserverbundbauteile 1, 2, 3 über einen vorbestimmten zweiten Zeitraum t2 einer zweiten Temperatur T2 und einer zweiten relativen Luftfeuchte ϕ2 ausgesetzt. Die zweite relative Luftfeuchte ϕ2 ist hierbei unterschiedlich zu der ersten relativen Luftfeuchte ϕ1, insbesondere größer als diese, wobei bevorzugt auch die zweite Temperatur T2 unterschiedlich zu der ersten Temperatur T1 ist, wie dies beispielhaft in Fig. 3 gezeigt ist. Fig. 3 zeigt beispielhaft einen Ermittlungszyklus C, bei dem die zweite Temperatur T2 kleiner ist als die erste Temperatur T1. Bevorzugt liegt die zweite Temperatur T2 zwischen 40 Grad Celsius und 200 Grad Celsius und die zweite relative Luftfeuchte ϕ2 liegt zwischen größer 50 Prozent und kleiner oder gleich 100 Prozent. Beispielsweise beträgt die zweite Temperatur T2 bevorzugt 100 Grad Celsius und die zweite relative Luftfeuchte ϕ2 liegt bei annähernd 100 Prozent, beispielsweise 97 Prozent. Auch ist bei dem in Fig. 3 gezeigten Beispiel der zweite Zeitraum t2 länger als der erste Zeitraum t1. Der erste und der zweite Zeitraum t1, t2 können jedoch auch gleich lang sein. Der Schritt M3.4 entspricht einer zweiten Wärmebehandlung der Referenzfaserverbundbauteile 1, 2, 3, bei der ein von dem Gesamtfeuchtigkeitsgehalt der Referenzfaserverbundbauteile 1, 2, 3 abhängiger Diffusionsprozess angeregt wird.

Bei dem in Fig. 3 beispielhaft dargestellten Ermittlungszyklus werden die Referenzbauteile 1, 2, 3 von der Ausgangstemperatur T0 auf die zweite Temperatur T2 erwärmt und anschließend wieder auf die Ausgangstemperatur T0 abgekühlt. Die zweite Temperatur T2 kann auch direkt ausgehend von der ersten Temperatur T1 eingestellt werden.

In Fig. 3 sind sämtliche Temperaturänderung zwischen der Ausgangstemperatur T0, der ersten Temperatur T1 und der zweiten Temperatur aus Gründen der Übersichtlichkeit als unendlich schnell ablaufend dargestellt. In der Realität laufen diese Temperaturänderungen selbstverständlich mit einer endlichen Geschwindigkeit ab.

Im nächsten Schritt M3.5 erfolgt eine Aufnahme von zweiten Messinfrarotspektren an den Oberflächen 1a, 2a, 3a der Referenzfaserverbundbauteile 1, 2, 3 mittels FTIR-Messungen. Dies erfolgt auf dieselbe Weise, wie z.B. für den Schritt M3.3 des Ermittlungszyklus C beschrieben wurde. In einem weiteren Schritt M3.6 des Ermittlungszyklus C erfolgt eine Bestimmung von zweiten, geänderten Oberflächenfeuchtegehalten der Referenzfaserverbundbauteile 1, 2, 3 aufgrund der zweiten Messinfrarotspektren anhand des spektrumsbasierten Vorhersagemodells für den Feuchtegehalt. Dies erfolgt auf dieselbe Weise, wie z.B. für den Schritt M3.4 des Ermittlungszyklus C beschrieben wurde.

Wie Fig. 5 beispielhaft zeigt, erfolgt während der zweiten Wärmebehandlung jeweils ein Anstieg der gemessenen Oberflächenfeuchten der Referenzbauteile 1, 2, 3. Insbesondere liegen die aus dem spektrumsbasierten Vorhersagemodell auf Basis der Messung #3 ermittelten zweiten Oberflächenfeuchten der Referenzbauteile 1, 2, 3 über den auf Basis der Messung #2 ermittelten Oberflächenfeuchten. Diese Änderung der Oberflächenfeuchte ist abhängig von Gesamtfeuchte des Referenzbauteils und der zweiten Temperatur T2, der zweiten relativen Luftfeuchtigkeit ϕ2 sowie der Dauer des zweiten Zeitraums t2.

Der vorbeschriebene Ermittlungszyklus C kann mehrmals wiederholt werden, wie dies in Fig. 1 schematisch gezeigt ist. In Fig. 3 ist beispielhaft ein Ermittlungszyklus C gezeigt, welcher einmal wiederholt wird. Dementsprechend zeigt Fig. 5 ein Referenzdiffusionsverhalten D_ref1, D_ref2, D_ref3 der jeweiligen Referenzbauteile 1, 2, 3, welches die Änderungen der Oberflächenfeuchten aus zwei Ermittlungszyklen umfasst.

Das im Ermittlungszyklus C ermittelte Diffusionsverhalten D_ref1, D_ref2, D_ref3 kann in Form der ermittelten Oberflächenfeuchtegehalten in dem Datenspeicher 32 der Analysevorrichtung 30 abgelegt sein.

Nach Beendigung des Ermittlungszyklus wird in einem vierten Schritt M4 des Verfahrens ein diffusionsbasiertes Vorhersagemodells für den Feuchtegehalt gebildet. Dies erfolgt durch Analyse des Referenzdiffusionsverhaltens D_ref1, D_ref2, D_ref3 mittels eines zweiten Regressionsverfahrens. Insbesondere können die Referenzfeuchten w_ref1, w_ref2, w_ref3 der Referenzbauteile den in Fig. 5 beispielhaft gezeigten Verläufen in Abhängigkeit der ersten und zweiten Temperatur T1, T2, der ersten und zweiten relativen Luftfeuchtigkeit ϕ1, ϕ2 sowie der Dauer des ersten und zweiten Zeitraums t1, t2 zugeordnet werden. Das diffusionsbasierte Vorhersagemodell gibt somit den Referenzfeuchtegehalt w_ref1, w_ref2, w_ref3 als Funktion von Parametern des Referenzdiffusionsverhaltens an.

Das diffusionsbasierte Vorhersagemodell für den Feuchtegehalt kann ebenfalls mit Hilfe der Datenverarbeitungseinrichtung 31 erstellt und in dem Datenspeicher 32 der Analysevorrichtung 30 abgelegt werden.

Im nächsten Schritt M5 des Verfahrens M wird ein tatsächliches Diffusionsverhalten D100 des Faserverbundbauteils 100, dessen Feuchtegehalt bestimmt werden soll, ermittelt. Hierzu wird der für die Referenzbauteile 1, 2, 3 durchgeführte Ermittlungszyklus C genauso oft und unter gleichen Bedingungen an dem Faserverbundbauteil 100 durchgeführt, wie dieser für die Referenzbauteile 1, 2, 3 durchgeführt wurde. Insbesondere wird jeweils die erste und die zweite Wärmebehandlung an dem Faserverbundbauteil 100 durchgeführt und anschließend jeweils ein Messinfrarotspektrum an der Oberfläche 100a des Faserverbundbauteils 100 aufgenommen und aus diesem anhand des spektrumsbasierten Vorhersagemodells die Oberflächenfeuchte des Faserverbundbauteils 100 bestimmt. Auf diese Weise wird ein tatsächliches Diffusionsverhalten D100 des Faserverbundbauteils 100 ermittelt. Fig. 6 zeigt beispielhaft das mittels zweimaliger Durchführung des Ermittlungszyklus C ermittelte tatsächliche Diffusionsverhalten D100 des Faserverbundbauteils 100.

Die das tatsächliche Diffusionsverhalten D100 beschreibenden Daten, insbesondere die ermittelten Oberflächenfeuchten des Faserverbundbauteils 100 können in dem Datenspeicher 32 der Analyseeinrichtung 30 abgelegt werden.

Im letzten Schritt M5 des Verfahrens M erfolgt eine Bestimmung des tatsächlichen Feuchtegehalts des Faserverbundbauteils 100 aufgrund des tatsächlichen Diffusionsverhaltens D100 des Faserverbundbauteils 100 anhand des diffusionsbasierten Vorhersagemodells. Hierbei wird das ermittelte tatsächliche Diffusionsverhalten D100 des Faserverbundbauteils 100 als Eingangsgröße für den durch das diffusionsbasierte Vorhersagemodell gegebenen funktionalen Zusammenhang zwischen dem Diffusionsverhalten und dem Feuchtegehalt verwendet. Das diffusionsbasierte Vorhersagemodell gibt als Ergebnis dann den Feuchtegehalt des Faserverbundbauteils 100 an.

Die Bestimmung des Feuchtegehalts mit Hilfe des diffusionsbasierten Vorhersagemodells kann ebenfalls durch die Datenverarbeitungseinrichtung 31 der Analysevorrichtung 30 erfolgen. Hierzu werden die tatsächliche Diffusionsverhalten D100 beschreibenden Daten, welche in dem Datenspeicher 32 abgelegt wurden, in der Datenverarbeitungseinrichtung 31 hinsichtlich bestimmter Parameter analysiert und das Ergebnis dieser Analyse als Input für das im Datenspeicher 32 abgelegte diffusionsbasierte Vorhersagemodell verwendet. Der ermittelte Feuchtegehalt des Faserverbundbauteils 100 kann wiederum in dem Datenspeicher 32 der Analysevorrichtung 30 abgelegt und/oder an einer Anzeigevorrichtung (nicht gezeigt) dargestellt werden.

In Fig. 7 ist schematisch eine Glocke 10 zur Durchführung des Ermittlungszyklus C gezeigt. Die Glocke 10 weist ein Gehäuse 11, eine Heizeinrichtung 12, ein Thermometer 13, sowie eine Gaszuleitung 14 und eine Gasableitung 15 auf. Wie in Fig. 7 gezeigt ist, definiert das Gehäuse 11 eine einseitig offene Kavität 16. Die Heizeinrichtung 12 kann insbesondere als Infrarotheizstrahler ausgeführt sein, welcher an einer Deckenwandung des Gehäuses 11 angeordnet und dessen Abstrahlungsrichtung der Öffnung der Kavität 16 zugewandt ist. Das Thermometer 13 ist bevorzugt als Pyrometer ausgeführt und funktional mit der Heizeinrichtung 12 gekoppelt, wobei die ermittelte Temperatur zur Einstellung der Heizleistung der Heizeinrichtung 12 verwendet wird. Die Gaszuleitung 14 und die Gasableitung 15 münden jeweils in die Kavität 16.

Fig. 7 zeigt die Glocke 10 während der Durchführung der ersten Wärmebehandlung. Die Glocke 10 ist hierbei mit der Öffnung der Kavität 16 auf die Oberfläche 1a, 2a, 3a des jeweiligen Referenzbauteils 1, 2, 3 bzw. die Oberfläche 100a des Faserverbundbauteils 100 aufgesetzt. Das Gehäuse 11 der Glocke 10 und die Oberfläche 1a, 2a, 3a des jeweiligen Referenzbauteils 1, 2, 3 bzw. die Oberfläche 100a des Faserverbundbauteils 100 begrenzen somit einen Arbeitsraum 17. In diesen wird über die Gaszuleitung 14 ein Luftmassenstrom der Temperatur T1 und der ersten relativen Luftfeuchtigkeit ϕ1 eingeleitet. Die Heizeinrichtung 12 stellt auf der Oberfläche 1a, 2a, 3a des jeweiligen Referenzbauteils 1, 2, 3 bzw. der Oberfläche 100a des Faserverbundbauteils 100 die erste Temperatur T1 ein. Über die Gasableitung 15 wird die Luft wieder aus dem Arbeitsraum 17 abgeleitet. Die zweite Wärmebehandlung kann auf analoge Weise durchgeführt werden, wobei hierbei die zweite Temperatur T2 und die zweit relative Luftfeuchtigkeit ϕ2 eingestellt werden.

Fig. 9 zeigt schematisch ein Messsystem 200 zur Messung der Feuchtigkeit des Faserverbundbauteils 100. Das Messsystem 200 weist die Glocke 10, das Spektrometer 20 einen Computer 40 mit zumindest einem Prozessor 41 sowie einen nicht-flüchtigen computerlesbaren Speicher 50 auf. Der Computer 40 kann insbesondere auch die Analysevorrichtung 30 aufweisen. Die Glocke 10, das Spektrometer 20 sind funktional an den Computer 40 gekoppelt, insbesondere sind von dem Computer erzeugte Steuerkommandos zur Steuerung der Komponenten der Glocke 10 und des Spektrometers 20 an diese und Signale, insbesondere Messsignale von der Glocke 10 bzw. dem Spektrometer 20 an den Computer 40 übertragbar. Der Computer 40 und der Speicher 50 sind geeignet, die Aufgaben der Analysevorrichtung 30 zu übernehmen oder diese auszubilden. In dem Speicher 50 ist ein Programm 51 gespeichert, das dazu ausgelegt ist, den Computer 40 zur Ausführung des voranstehend beschriebenen Verfahrens M zu veranlassen.

Obwohl die vorliegende Erfindung vorstehend anhand von Ausführungsbeispielen exemplarisch erläutert wurde, ist sie darauf nicht beschränkt, sondern auf vielfältige Weise modifizierbar. Insbesondere sind auch Kombinationen der voranstehenden Ausführungsbeispiele denkbar.

### BEZUGSZEICHENLISTE

- 1: erstes Referenzfaserverbundbauteil
- 1a: Oberfläche des ersten Referenzfaserverbundbauteils
- 2: zweites Referenzfaserverbundbauteil
- 2a: Oberfläche des zweiten Referenzfaserverbundbauteils
- 3: drittes Referenzfaserverbundbauteil
- 3a: Oberfläche des dritten Referenzfaserverbundbauteils
- 10: Glocke
- 11: Gehäuse
- 12: Heizeinrichtung
- 13: Thermometer
- 14: Gaszuleitung
- 15: Gasableitung
- 16: Kavität
- 17: Arbeitsraum
- 20: Spektrometer
- 21: IR-Strahlung
- 30: Analysevorrichtung
- 31: Datenverarbeitungseinrichtung
- 32: Datenspeicher
- 40: Computer
- 50: nicht-flüchtiger Speicher
- 51: Programm
- 100: Faserverbundbauteil
- 100a: Oberfläche des Faserverbundbauteils
- 200: Messsystem
- C: Ermittlungszyklus
- D_ref1: Referenzdiffusionsverhalten des ersten Referenzfaserverbundbauteils
- D_ref2: Referenzdiffusionsverhalten des zweiten Referenzfaserverbundbauteils
- D_ref3: Referenzdiffusionsverhalten des dritten Referenzfaserverbundbauteils
- E1: Absorptionsmaximum
- E2: Absorptionsmaximum
- M: Verfahren
- M1: Verfahrensschritt
- M2: Verfahrensschritt
- M3: Verfahrensschritt
- M3.1: Schritt des Ermittlungszyklus
- M3.2: Schritt des Ermittlungszyklus
- M3.3: Schritt des Ermittlungszyklus
- M3.4: Schritt des Ermittlungszyklus
- M3.5: Schritt des Ermittlungszyklus
- M3.6: Schritt des Ermittlungszyklus
- M4: Verfahrensschritt
- M5: Verfahrensschritt
- M6: Verfahrensschritt
- S1: Referenzinfrarotspektrum des ersten Referenzfaserverbundbauteils
- S2: Referenzinfrarotspektrum des zweiten Referenzfaserverbundbauteils
- S3: Referenzinfrarotspektrum des dritten Referenzfaserverbundbauteils
- Sm1: erstes Messinfrarotspektrum des ersten Referenzfaserverbundbauteils
- Sm2: erstes Messinfrarotspektrum des zweiten Referenzfaserverbundbauteils
- Sm3: erstes Messinfrarotspektrum des dritten Referenzfaserverbundbauteils
- T1: erste Temperatur
- t1: erster Zeitraum
- T2: zweite Temperatur
- t2: zweiter Zeitraum
- w_ref1: Referenzfeuchtegehalt des ersten Referenzfaserverbundbauteils
- w_ref2: Referenzfeuchtegehalt des zweiten Referenzfaserverbundbauteils
- w_ref3: Referenzfeuchtegehalt des dritten Referenzfaserverbundbauteils
- ϕ1: erste relative Luftfeuchte
- ϕ2: zweite relative Luftfeuchte

## Patentansprüche

1. Verfahren (M) zur Bestimmung des Gesamtfeuchtegehalts eines Faserverbundbauteils (100) mit folgenden Verfahrensschritten:
Aufnahme (M1) von ersten Referenzinfrarotspektren (S1; S2; S3) an Oberflächen (1a; 2a; 3a) von Referenzfaserverbundbauteilen (1; 2; 3), welche jeweils einen definierten Referenzfeuchtegehalt (w_ref1, w_ref2, w_ref3) aufweisen, mittels FTIR-Messungen;
Bilden (M2) eines spektrumsbasierten Vorhersagemodells für einen Oberflächenfeuchtegehalt durch Analyse der ersten Referenzinfrarotspektren (S1; S2; S3) mittels eines ersten Regressionsverfahrens;
Ermitteln (M3) eines Referenzdiffusionsverhaltens (D_refl; D_ref2; D_ref3) der Referenzfaserverbundbauteile (1; 2; 3) mittels mindestens einmaliger Durchführung des folgenden Ermittlungszyklus (C):
Aussetzen (M3.1) der Oberflächen (1a; 2a; 3a) der Referenzfaserverbundbauteile (1; 2; 3) einer ersten Temperatur (T1) und einer ersten relativen Luftfeuchte (ϕ1) über einen vorbestimmten ersten Zeitraum (t1);
Aufnahme (M3.2) von ersten Messinfrarotspektren (Sm1; Sm2; Sm3) an den Oberflächen (1a; 2a; 3a) der Referenzfaserverbundbauteile (1; 2; 3) mittels FTIR-Messungen;
Bestimmung (M3.3) von ersten Oberflächenfeuchtegehalten der Referenzfaserverbundbauteile (1; 2; 3) aufgrund der ersten Messinfrarotspektren (Sm1) anhand des spektrumsbasiert Vorhersagemodells;
Aussetzen (M3.4) der Oberflächen (1a; 2a; 3a) der Referenzfaserverbundbauteile (1; 2; 3) einer zweiten Temperatur (T2) und einer zweiten relativen Luftfeuchte (ϕ2) über einen vorbestimmten zweiten Zeitraum (t2);
Aufnahme (M3.5) von zweiten Messinfrarotspektren an den Oberflächen (1a; 2a; 3a) der Referenzfaserverbundbauteile (1; 2; 3) mittels FTIR-Messungen; und
Bestimmung (M3.6) von zweiten Oberflächenfeuchtegehalten der Referenzfaserverbundbauteile (1; 2; 3) aufgrund der zweiten Messinfrarotspektren anhand des spektrumsbasierten Vorhersagemodells;
Bilden (M4) eines diffusionsbasierten Vorhersagemodells für den Gesamtfeuchtegehalt durch Analyse des Referenzdiffusionsverhaltens (D_refl; D_ref2; D_ref3) mittels eines zweiten Regressionsverfahrens;
Ermitteln (M5) eines tatsächlichen Diffusionsverhaltens (D100) des Faserverbundbauteils (100) mittels mindestens einmaliger Durchführung des Ermittlungszyklus (C) an dem Faserverbundbauteil (100); und
Bestimmen (M5) des tatsächlichen Gesamtfeuchtegehalts des Faserverbundbauteils (100) aufgrund des tatsächlichen Diffusionsverhaltens (D100) des Faserverbundbauteils (100) anhand des diffusionsbasierten Vorhersagemodells.

2. Verfahren (M) nach Anspruch 1, wobei das erste Regressionsverfahren eine multivariate Analyse des jeweiligen Referenzinfrarotspektrums (S1; S2; S3) umfasst.

3. Verfahren (M) nach Anspruch 1 oder 2, wobei das zweite Regressionsverfahren eine multivariate Analyse des Referenzdiffusionsverhaltens (D_ref1; D_ref2; D_ref3) umfasst.

4. Verfahren (M) nach Anspruch 2 oder 3, wobei die multivariate Analyse die Durchführung eines Partial Least Squares, PLS, Algorithmus umfasst.

5. Verfahren (M) nach einem der voranstehenden Ansprüche, wobei bei dem Ermittlungszyklus (C) nach der Bestimmung (M3.3) der ersten Oberflächenfeuchtegehalte der Referenzfaserverbundbauteile (1; 2; 3) die Schritte des Aussetzens (M3.1) der Oberflächen der Referenzfaserverbundbauteile (1; 2; 3) der ersten Temperatur (T1) und der ersten relativen Luftfeuchte (ϕ1), der Aufnahme (M3.2) der ersten Messinfrarotspektren (Sm1; Sm2; Sm3) und der Bestimmung (M3.3) der ersten Oberflächenfeuchtegehalte der Referenzfaserverbundbauteile (1; 2; 3) wiederholt werden.

6. Verfahren (M) nach einem der voranstehenden Ansprüche, wobei die Referenzfeuchtegehalte (w_ref1, w_ref2, w_ref3) der Referenzfaserverbundbauteile (1; 2; 3) zwischen Null Gewichtsprozent und 3 Gewichtsprozent liegen.

7. Verfahren (M) nach einem der voranstehenden Ansprüche, wobei die erste Temperatur (T1) in einem Bereich zwischen 90 Grad Celsius und 200 Grad Celsius liegt und die erste relative Luftfeuchte (ϕ1) der ersten in einem Bereich zwischen 0 Prozent und 50 Prozent liegt.

8. Verfahren (M) nach einem der voranstehenden Ansprüche, wobei der erste Zeitraum (t1) zwischen 0,5 Minuten und 30 Minuten beträgt.

9. Verfahren (M) nach einem der voranstehenden Ansprüche, wobei die zweite Temperatur (T2) in einem Bereich zwischen 40 Grad Celsius und 200 Grad Celsius liegt und die zweite relative Luftfeuchte (ϕ2) in einem Bereich zwischen größer 50 Prozent und kleiner oder gleich 100 Prozent liegt.

10. Verfahren (M) nach einem der voranstehenden Ansprüche, wobei der zweite Zeitraum (t2) zwischen 0,5 Minuten und 24 Stunden beträgt.

11. Verfahren (M) nach einem der voranstehenden Ansprüche, wobei die erste und die zweite Temperatur (Tl; T2) und die erste und die zweite relative Luftfeuchte (ϕ1; ϕ2) jeweils in einem Arbeitsraum (17) erzeugt werden, welcher durch eine an der Oberfläche (1a; 2a; 3a) des Referenzfaserverbundbauteils (1; 2; 3) oder an der Oberfläche (100a) des Faserverbundbauteils (100) anliegende Glocke (10) begrenzt wird.

12. Verfahren (M) nach Anspruch 11, wobei die Glocke (10) zur Aufnahme (M2.2) der ersten Messinfrarotspektren (Sm1; Sm2; Sm3) und zur Aufnahme (M2.5) der zweiten Messinfrarotspektren von der Oberfläche (1a; 2a; 3a) des Referenzfaserverbundbauteils (1; 2; 3) oder an der Oberfläche (100a) des Faserverbundbauteils (100) entfernt wird.

13. Computerlesbarer, nicht-flüchtiger Datenspeicher (50), welcher ein Programm (51) speichert, das dazu ausgelegt ist, einen Computer (40) zur Ausführung eines Verfahrens (M) nach einem der voranstehenden Ansprüche zu veranlassen.

14. Messsystem (200) zur Messung der Feuchtigkeit des Faserverbundbauteils (100) mit
einer Glocke (10) zur Einstellung einer ersten Temperatur (T1) und einer ersten relativen Luftfeuchtigkeit (ϕ1) sowie einer zweiten Temperatur (T2) und einer ersten relativen Luftfeuchtigkeit (ϕ2) an einer Oberfläche (100a) des Faserverbundbauteils (10);
einem Spektrometer (20);
einem Computer (40), an welchen die Glocke (10) und das Spektrometer (20) funktional gekoppelt sind; und
einem von dem Computer (40) lesbaren, nicht-flüchtiger Datenspeicher (50) nach Anspruch 13.

## Claims

1. Method (M) for determining the overall moisture content of a fibre composite component (100), including the following method steps:
recording (M1) first reference infrared spectra (S1; S2; S3) of surfaces (1a; 2a; 3a) of reference fibre composite components (1; 2; 3), which each have a defined reference moisture content (w_ref1, w_ref2, w_ref3), by means of FTIR measurements;
forming (M2) a spectrum-based prediction model for a surface moisture content by analysing the first reference infrared spectra (S1; S2; S3) by means of a first regression method;
establishing (M3) a reference diffusion behaviour (D_ref1; D_ref2; D_ref3) of the reference fibre composite components (1; 2; 3) by implementing the following establishment cycle (C) at least once:
exposing (M3.1) the surfaces (1a; 2a; 3a) of the reference fibre composite components (1; 2; 3) to a first temperature (T1) and a first relative humidity (ϕ1) over a predetermined first period of time (t1);
recording (M3.2) first measurement infrared spectra (Sm1; Sm2; Sm3) of the surfaces (1a; 2a; 3a) of the reference fibre composite components (1; 2; 3) by means of FTIR measurements;
determining (M3.3) first surface moisture contents of the reference fibre composite components (1; 2; 3) on the basis of the first measurement infrared spectra (Sm1) using the spectrum-based prediction model;
exposing (M3.4) the surfaces (1a; 2a; 3a) of the reference fibre composite components (1; 2; 3) to a second temperature (T2) and a second relative humidity (ϕ2) over a predetermined second period of time (t2);
recording (M3.5) second measurement infrared spectra of the surfaces (1a; 2a; 3a) of the reference fibre composite components (1; 2; 3) by means of FTIR measurements; and determining (M3.6) second surface moisture contents of the reference fibre composite components (1; 2; 3) on the basis of the second measurement infrared spectra using the spectrum-based prediction model;
forming (M4) a diffusion-based prediction model for the overall moisture content by analysing the reference diffusion behaviour (D_ref1; D_ref2; D_ref3) by means of a second regression method;
establishing (M5) an actual diffusion behaviour (D100) of the fibre composite component (100) by implementing the establishment cycle (C) at least once on the fibre composite component (100); and
determining (M5) the actual overall moisture content of the fibre composite component (100) on the basis of the actual diffusion behaviour (D100) of the fibre composite component (100) using the diffusion-based prediction model.

2. Method (M) according to Claim 1, wherein the first regression method comprises a multivariate analysis of the respective reference infrared spectrum (S1; S2; S3).

3. Method (M) according to Claim 1 or 2, wherein the second regression method comprises a multivariate analysis of the reference diffusion behaviour (D_ref1; D_ref2; D_ref3).

4. Method (M) according to Claim 2 or 3, wherein the multivariate analysis comprises the implementation of a partial least squares, PLS, algorithm.

5. Method (M) according to any one of the preceding claims, wherein, after determining (M3.3) the first surface moisture contents of the reference fibre composite components (1; 2; 3) in the establishment cycle (C), the steps of exposing (M3.1) the surfaces of the reference fibre composite components (1; 2; 3) to the first temperature (T1) and the first relative humidity (ϕ1) , recording (M3.2) the first measurement infrared spectra (Sm1; Sm2; Sm3) and determining (M3.3) the first surface moisture contents of the reference fibre composite components (1; 2; 3) are repeated.

6. Method (M) according to any one of the preceding claims, wherein the reference moisture contents (w_ref1, w_ref2, w_ref3) of the reference fibre composite components (1; 2; 3) lie between zero percent by weight and 3 percent by weight.

7. Method (M) according to any one of the preceding claims, wherein the first temperature (T1) lies in a range between 90 degrees Celsius and 200 degrees Celsius and the first relative humidity (ϕ1) of the first lies in a range between 0 percent and 50 percent.

8. Method (M) according to any one of the preceding claims, wherein the first period of time (t1) is between 0.5 minutes and 30 minutes.

9. Method (M) according to any one of the preceding claims, wherein the second temperature (T2) lies in a range between 40 degrees Celsius and 200 degrees Celsius and the second relative humidity (ϕ2) lies in a range between greater than 50 percent and less than or equal to 100 percent.

10. Method (M) according to any one of the preceding claims, wherein the second period of time (t2) is between 0.5 minutes and 24 hours.

11. Method (M) according to any one of the preceding claims, wherein the first and the second temperature (T1; T2) and the first and the second relative humidity (ϕ1; ϕ2) are each produced in a work chamber (17), which is delimited by a dome (10) that rests against the surface (1a; 2a; 3a) of the reference fibre composite component (1; 2; 3) or against the surface (100a) of the fibre composite component (100).

12. Method (M) according to Claim 11, wherein the dome (10) is removed for recording (M2.2) the first measurement infrared spectra (Sm1; Sm2; Sm3) and for recording (M2.5) the second measurement infrared spectra of the surface (1a; 2a; 3a) of the reference fibre composite component (1; 2; 3) or of the surface (100a) of the fibre composite component (100).

13. Computer-readable, non-volatile data memory (50), which stores a program (51) designed to prompt a computer (40) to carry out a method (M) according to any one of the preceding claims.

14. Measurement system (200) for measuring the moisture of the fibre composite component (100), comprising
a dome (10) for setting a first temperature (T1) and a first relative humidity (ϕ1) and also a ssecond temperature (T2) and a first relative humidity (ϕ2) on a surface (100a) of the fibre composite component (10);
a spectrometer (20);
a computer (40), to which the dome (10) and the spectrometer (20) are functionally coupled; and
a non-volatile data memory (50) according to Claim 13 that is readable by the computer (40).

## Revendications

1. Procédé (M) pour identifier la teneur en humidité totale d'un élément structural composite à base de fibres (100), comprenant les étapes de procédé suivantes :
relevé (M1) de premiers spectres infrarouges de référence (S1 ; S2 ; S3) au niveau de surfaces (1a ; 2a ; 3a) d'éléments structuraux composites à base de fibres de référence (1 ; 2 ; 3), lesquels présentent respectivement une teneur en humidité de référence (w_ref1, w_ref2, w_ref3) définie au moyen de mesures FTIR ;
formation (M2) d'un modèle de prévision basé sur le spectre pour une teneur en humidité de surface par analyse des premiers spectres infrarouges de référence (S1 ; S2 ; S3) au moyen d'un premier procédé de régression ;
détermination (M3) d'un comportement de diffusion de référence (D_ref1 ; D_ref2 ; D_ref3) des éléments structuraux composites à base de fibres de référence (1 ; 2 ; 3) en exécutant au moins une fois le cycle de détermination (C) suivant :
exposition (M3.1) des surfaces (1a ; 2a ; 3a) des éléments structuraux composites à base de fibres de référence (1 ; 2 ; 3) à une première température (T1) et une première humidité de l'air relative (ϕ1) sur une première période (t1) prédéterminée ;
relevé (M3.2) de premiers spectres infrarouges de mesure (Sm1 ; Sm2 ; Sm3) au niveau des surfaces (1a ; 2a ; 3a) des éléments structuraux composites à base de fibres de référence (1 ; 2 ; 3) au moyen de mesures FTIR ;
identification (M3.3) de premières teneurs en humidité de surface des éléments structuraux composites à base de fibres de référence (1 ; 2 ; 3) sur la base des premiers spectres infrarouges de mesure (Sm1) à l'aide du modèle de prévision basé sur le spectre ;
exposition (M3.4) des surfaces (1a ; 2a ; 3a) des éléments structuraux composites à base de fibres de référence (1 ; 2 ; 3) à une deuxième température (T2) et une deuxième humidité de l'air relative (ϕ2) sur une deuxième période (t2) prédéterminée ;
relevé (M3.5) de deuxièmes spectres infrarouges de mesure au niveau des surfaces (1a ; 2a ; 3a) des éléments structuraux composites à base de fibres de référence (1 ; 2 ; 3) au moyen de mesures FTIR ; et
identification (M3.6) de deuxièmes teneurs en humidité de surface des éléments structuraux composites à base de fibres de référence (1 ; 2 ; 3) sur la base des deuxièmes spectres infrarouges de mesure à l'aide du modèle de prévision basé sur le spectre ;
formation (M4) d'un modèle de prévision basé sur la diffusion pour la teneur en humidité totale par analyse du comportement de diffusion de référence (D_ref1 ; D_ref2 ; D_ref3) au moyen d'un deuxième procédé de régression ;
détermination (M5) d'un comportement de diffusion (D100) réel de l'élément structural composite à base de fibres (100) en exécutant au moins une fois le cycle de détermination (C) sur l'élément structural composite à base de fibres (100) ; et
identification (M5) de la teneur en humidité totale réelle de l'élément structural composite à base de fibres (100) sur la base du comportement de diffusion (D100) réel de l'élément structural composite à base de fibres (100) à l'aide du modèle de prévision basé sur la diffusion.

2. Procédé (M) selon la revendication 1, le premier procédé de régression comprenant une analyse à variables multiples du spectre infrarouge de référence (S1 ; S2 ; S3) respectif.

3. Procédé (M) selon la revendication 1 ou 2, le deuxième procédé de régression comprenant une analyse à variables multiples du comportement de diffusion de référence (D_ref1 ; D_ref2 ; D_ref3).

4. Procédé (M) selon la revendication 2 ou 3, l'analyse à variables multiples comprenant un algorithme des moindres carrés partiels, PLS.

5. Procédé (M) selon l'une des revendications précédentes, selon lequel, lors du cycle de détermination (C) les étapes d'exposition (M3.1) des surfaces des éléments structuraux composites à base de fibres de référence (1 ; 2 ; 3) à la première température (T1) et à la première humidité de l'air relative (ϕ1), de relevé (M3.2) des premiers spectres infrarouges de mesure (Sm1 ; Sm2 ; Sm3) et de détermination (M3.3) des premières teneurs en humidité de surface des éléments structuraux composites à base de fibres de référence (1 ; 2 ; 3) sont répétées après l'identification (M3.3) des premières teneurs en humidité de surface des éléments structuraux composites à base de fibres de référence (1 ; 2 ; 3).

6. Procédé (M) selon l'une des revendications précédentes, les teneurs en humidité de référence (w_ref1, w_ref2, w_ref3) des éléments structuraux composites à base de fibres de référence (1 ; 2 ; 3) étant comprises entre zéro pourcent massique et 3 pourcents massiques.

7. Procédé (M) selon l'une des revendications précédentes, la première température (T1) étant comprise dans une plage entre 90 degrés Celsius et 200 degrés Celsius et la première humidité de l'air relative (ϕ1) de la première étant comprise dans une plage entre 0 pourcent et 50 pourcents.

8. Procédé (M) selon l'une des revendications précédentes, la première période (t1) étant comprise entre 0,5 minute et 30 minutes.

9. Procédé (M) selon l'une des revendications précédentes, la deuxième température (T2) étant comprise dans une plage entre 40 degrés Celsius et 200 degrés Celsius et la deuxième humidité de l'air relative (ϕ2) étant comprise dans une plage entre plus de 50 pourcents et 100 pourcents ou moins.

10. Procédé (M) selon l'une des revendications précédentes, la deuxième période (t2) étant comprise entre 0,5 minute et 24 heures.

11. Procédé (M) selon l'une des revendications précédentes, la première et la deuxième température (T1 ; T2) ainsi que la première et la deuxième humidité de l'air relative (ϕ1 ; ϕ2) étant respectivement produites dans un espace de travail (17) qui est délimité par une cloche (10) qui repose sur la surface (1a ; 2a ; 3a) de l'élément structural composite à base de fibres de référence (1 ; 2 ; 3) ou sur la surface (100a) de l'élément structural composite à base de fibres (100).

12. Procédé (M) selon la revendication 11, la cloche (10) étant éloignée de la surface (1a ; 2a ; 3a) de l'élément structural composite à base de fibres de référence (1 ; 2 ; 3) ou de la surface (100a) de l'élément structural composite à base de fibres (100) en vue du relevé (M2.2) des premiers spectres infrarouges de mesure (Sm1 ; Sm2 ; Sm3) et du relevé (M2.5) des deuxièmes spectres infrarouges de mesure.

13. Mémoire de données (50) non volatile lisible par ordinateur, laquelle mémorise un programme (51) qui est conçu pour amener un ordinateur (40) à mettre en oeuvre un procédé (M) selon l'une des revendications précédentes.

14. Système de mesure (200) destiné à mesurer l'humidité d'un élément structural composite à base de fibres (100), comprenant
une cloche (10) destinée à régler une première température (T1) et une première humidité de l'air relative (ϕ1) ainsi qu'une deuxième température (T2) et une première humidité de l'air relative (ϕ2) au niveau d'une surface (100a) de l'élément structural composite à base de fibres (10) ;
un spectromètre (20) ;
un ordinateur (40) auquel la cloche (10) et le spectromètre (20) sont connectés de manière fonctionnelle ; et
une mémoire de données (50) non volatile selon la revendication 13, lisible par l'ordinateur (40).
